Europäisches Patentamt

European Patent Office

Office européen des brevets

(11) Veröffentlichungsnummer: **0 202 403**
**A1**

## (12) EUROPÄISCHE PATENTANMELDUNG

(21) Anmeldenummer: **86102775.3**

(22) Anmeldetag: **03.03.86**

(51) Int. Cl.4: **C07C 45/46** , **C07C 49/788**

(30) Priorität: **24.05.85 DE 3518668**

(43) Veröffentlichungstag der Anmeldung:
**26.11.86 Patentblatt 86/48**

(84) Benannte Vertragsstaaten:
**DE FR GB IT NL**

(71) Anmelder: **Rütgerswerke Aktiengesellschaft**
**Mainzer Landstrasse 217**
**D-6000 Frankfurt a.Main 1(DE)**

(72) Erfinder: **Kajetanczyk, Franz**
**Brauckweg 13**
**D-4620 Castrop-Rauxel(DE)**
Erfinder: **Steinbach, Rainer, Dr.**
**Bochumer Strasse 65**
**D-4620 Castrop-Rauxel(DE)**
Erfinder: **Ruppert, Ingo, Dr.**
**Nordstrasse 7**
**D-5300 Bonn(DE)**
Erfinder: **Schlich, Klaus**
**Falterer Pfad 13**
**D-5480 Remagen(DE)**

(54) Verfahren zur Acylierung von 2-Alkylnaphthalinen.

(57) 2-Alkylnaphthaline werden selektiv in 6-Stellung acyliert, wenn sie in einem aprotischen Lösemittel gelöst und unter katalytischer Wirkung von $BF_3$ mit Carbonsäureanhydrid oder -fluorid umgesetzt werden.

EP 0 202 403 A1

## Verfahren zur Acylierung von 2-Alkylnaphthalinen

Die Erfindung betrifft ein neues Verfahren zur selek-tiven Acylierung von 2-Alkylnaphthalinen.

Es ist bekannt, alkylsubstituierte Aromaten durch Umsetzung mit Carbonsäure-halogeniden oder -anhydriden zu acylieren, wobei als Katalysatoren Friedel-Crafts-Katalysatoren verwendet werden.

Mittels Friedel-Crafts-Katalysatoren durchgeführte Reaktionen sind dafür bekannt, daß neben dem gewünschten Hauptprodukt relativ viele Nebenprodukte entstehen, was häufig zu gänzlich unerwünschten Harzbildungen führt. Friedel-Crafts-Reaktionen sind demnach wenig selektiv. Noch ausgeprägter ist dieser Nachteil, wenn man anstelle einkerniger aromatischer Verbindungen mehrkernige Aromaten acylieren will, da hier die Substitutionsmöglichkeiten noch größer sind.

Andererseits wird bei der Acylierung von 2-Alkylnaphthalinen eine möglichst hohe Ausbeute an 2-Alkyl-6-acylnaphthalin benötigt, die vorteilhaft für die Weiterverarbeitung von 2,6-Dicarbonsäurederivaten ist, wobei es zusätzlich notwendig ist, daß sich die weiteren entstehenden Reaktionsprodukte leicht vom Hauptprodukt abtrennen lassen.

Aufgabe der Erfindung ist daher ein Verfahren zur Acylierung von 2-Alkylnaphthalinen, bei dem in hoher Ausbeute und Selektivität 2-Alkyl-6-acylnaphthalin entsteht.

Die Aufgabe wird gelöst durch ein Verfahren gemäß der Ansprüche 1 bis 4.

Es wurde gefunden, daß 2-Alkylnaphthaline nahezu quantitativ umgesetzt werden können, wobei in hoher Selektivität 2-Alkyl-6-acylnaphthaline entstehen, wenn die 2-Alkylnaphthaline in einem polaren aprotischen Lösemittel gelöst, in diese Lösung $BF_3$ eingeleitet und bei einer Temperatur im Bereich von -50 bis +50°C mit Carbonsäureanhydrid oder Carbonsäurefluorid zugegeben wird.

Als 2-Alkylnaphthaline können alle Naphthalinderivate eingesetzt werden, die in 2-Stellung mit einer Alkyl-oder Cycloalkylgruppe substituiert sind. Im Hinblick auf die geplante Weiterverarbeitung, die Oxidation des Acylierungsproduktes wird aus wirtschaftlichen Gründen nur 2-Methyl-, 2-Ethyl-, 2-Propyl-oder 2-Isopropylnaphthalin eingesetzt.

Polare, aprotische Lösemittel sind z.B. Methylenchlorid, Chloroform, Tetrachlorkohlenstoff, Dichlorethan, Schwefelkohlenstoff oder Nitrobenzol.

Acylierungskatalysator ist Bortrifluorid, das gasförmig in die Lösung eingebracht wird. Die spezielle katalytische Wirkung von Borfluorid ist insofern überraschend, als die bei Friedel-Crafts-Reaktionen üblicherweise eingesetzten $BF_3$-Addukte wie $BF_3$.HF, $BF_3$-Etherate oder $BF_3$-Alkoholate nicht die gewünschte selektive Acylierung katalysieren.

Bortrifluorid wird zweckmäßigerweise in doppelter äquimolarer Menge, bezogen auf das 2-Alkylnaphthalin, eingesetzt.

Die so hergestellte Lösung, die 2-Alkylnaphthalin und $BF_3$ enthält, wird auf eine Temperatur im Bereich von -50 bis + 50°C, bevorzugt im Bereich von -10 bis 20°C gebracht und während der ganzen Reaktionszeit in diesem Bereich gehalten.

Zu der Lösung wird Carbonsäureanhydrid oder Carbonsäurefluorid in äquimolarer Menge, bezogen auf das 2-Alkylnaphthalin, zugegeben, wobei die Reaktionstemperatur entweder durch Kühlung oder durch genau dosierte Zugabe des Acylierungsmittels gesteuert wird.

Als Carbonsäureanhydride oder -fluoride sind Anhydride der Essig-, Propion-oder Buttersäure, bevorzugt die der Essigsäure einsetzbar.

Nach erfolgter Umsetzung wird das Reaktionsgemisch in an sich bekannter Weise aufgearbeitet.

Beispiele

Beispiel 1

142 g (1 Mol) 2-Methylnaphthalin werden in 500 ml $CH_2Cl_2$ gelöst. In die auf 0°C gekühlte Lösung werden 136 g gasförmiges $BF_3$ eingeleitet und danach im Verlauf von 3 h 102 g (1 Mol) Acetanhydrid eingeleitet und dabei unter Rühren die Temperatur auf 0°C gehalten. Danach wird das Reaktionsgemisch vorsichtig auf etwa 1000 g Eis gegossen und die sich dann abtrennende organische Phase abgenommen und destillativ aufgearbeitet. Umsatz: 99 % des 2-Methylnaphthalins Ausbeute an 2-Methyl-6-acetylnaphthalin: 89 %, bezogen auf eingesetztes 2-Methylnaphthalin.

**Ansprüche**

1.Verfahren zur selektiven Acylierung von 2-Alkylnaphthalin,**dadurch gekennzeichnet**, daß das alkylsubstituierte Naphthalin in einem polaren, aprotischen Lösemittel gelöst, in diese Lösung $BF_3$ eingeleitet und bei einer Temperatur im Bereich von -

50 bis + 50°C Carbonsäureanhydrid oder Carbonsäurefluorid zugegeben wird.

2. Verfahren nach Anspruch 1,**dadurch gekennzeichnet** , daß die Umsetzung im Temperaturbereich von -10 bis + 20°C durchgeführt wird.

3.Verfahren nach Anspruch 1 und 2, **dadurch gekennzeichnet**, daß als Acylierungsmittel Acetanhydrid verwendet wird.

4. Verfahren zur Herstellung von 2-Methyl-6-acetyl-naphthalin,**dadurch gekennzeichnet**, daß 2-Methyl-naphthalin mittels eines Verfahrens gemäß Anspruch 1 acyliert wird.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | US-A-3 234 286 (F.R. LAWRENCE) <br> * Patentansprüche * | 1-4 | C 07 C  45/46 <br> C 07 C  49/788 |
| Y | US-A-2 463 742 (A.C. BYRNS) <br> * Spalte 1, Zeilen 25-28; Spalte 2, Zeilen 12-17; Patentansprüche * | 1-3 | |
| Y | GB-A-1 603 075 (SECRETARY OF STATE FOR DEFENCE) <br> * Seite 4, Beispiel 2 * | 1-4 | |
| Y | JOURNAL OF THE AMERICAN CHEMICAL SOCIETY, Band 49, 1984, Seiten 384-385, American Chemical Society, Gaston, Pa, US; J.A. HYATT et al.: "Acyl fluoride friedel-crafts reactions. regioselective synthesis of 3-acylacenaphthenes and 2-acyl-6-alkylnaphthalenes" <br> * Seiten 384-385 * | 1-4 | RECHERCHIERTE SACHGEBIETE (Int. Cl.4) <br><br> C 07 C  45/00 <br> C 07 C  49/00 |

Der vorliegende Recherchenbericht wurde für alle Patentansprüche erstellt.

| Recherchenort <br> DEN HAAG | Abschlußdatum der Recherche <br> 20-06-1986 | Prüfer <br> BONNEVALLE E.I.H. |
|---|---|---|